# EUROPEAN PATENT APPLICATION

(11) **EP 4 393 934 A1**
(43) Date of publication of application: **03.07.2024**
(21) Application number: 22861466.5
(22) Date of filing: 26.08.2022
(51) Int. Cl.: C07H 19/10

(54) **METHOD FOR PRODUCING CYTIDINE-5'-DIPHOSPHATE COMPOUND**

(30) Priority: 26.08.2021 WO PCT/JP2021/031429
(71) Applicant: Kyowa Hakko Bio Co., Ltd., Tokyo 100-8185 (JP)
(72) Inventor: MIYAZAKI Takumi, Tokyo 100-8185 (JP); FUKUMOTO Kazunari, Tokyo 100-8185 (JP)
(74) Representative: Vossius & Partner Patentanwälte Rechtsanwälte mbB
(86) International application number: PCT/JP2022/032235
(87) International publication number: WO 2023/027178

(57) **Abstract**

The present invention relates to a method for producing a cytidine-5'-diphosphate compound. The method includes: (1) a step of bringing a solution containing a cytidine-5'-diphosphate compound into contact with a basic ion exchange resin to adsorb the cytidine-5'-diphosphate compound onto the basic ion exchange resin, and (2) a step of obtaining an eluate by eluting the cytidine-5'-diphosphate compound adsorbed onto the basic ion exchange resin using an aqueous solution containing an organic acid having 2 or more carbon atoms. The cytidine-5'-diphosphate compound is a cytidine-5'-diphosphate compound having a cationic substituent.

## Description

### TECHNICAL FIELD

The present invention relates to a method for producing a cytidine-5'-diphosphate compound.

### BACKGROUND ART

An industrial production method for a cytidine-5'-diphosphate compound including cytidine-5'-diphosphate choline (hereinafter also abbreviated as CDP-choline) includes a chemical synthesis method, a fermentation method using microorganisms, and an enzyme method. In either method, since the method progresses through a reaction in which cytidine-5'-monophosphate (hereinafter also abbreviated as 5'-CMP) or a highly reactive derivative thereof is condensed with phosphocholine or phosphoethanolamine, 5'-CMP derived from a raw material or produced as a by-product exists as an impurity in the system. The cytidine-5'-diphosphate compound is hydrolyzed by heating, and 5'-CMP is produced as a by-product.

In related art, examples of a general method for purifying a cytidine-5'-diphosphate compound from a solution containing a cytidine-5'-diphosphate compound include column chromatography using an ion exchange resin. For example, Patent Literatures 1 to 3 disclose a method in which crude CDP-choline is adsorbed onto an anion exchange resin and then the adsorbed crude CDP-choline is eluted using extremely dilute formic acid or hydrochloric acid of about 0.005 mol/L to 0.10 mol/L.

Patent Literature 4 discloses a method using a sodium chloride aqueous solution as an eluent. As still another method, Patent Literature 5 discloses a method in which crude CDP-choline is passed through a weakly basic anion exchange resin and only an impurity 5'-CMP is selectively adsorbed and removed.

### CITATION LIST

### PATENT LITERATURE

Patent Literature 1: JPS48-86869A
Patent Literature 2: JPS47-18881A
Patent Literature 3: Indian Patent Application Publication No. 2012MUM02534
Patent Literature 4: China Patent Application Publication No. 101130797
Patent Literature 5: JPH6-31306B

### SUMMARY OF INVENTION

### TECHNICAL PROBLEM

In a process of purifying a cytidine-5'-diphosphate compound from a solution containing a cytidine-5'-diphosphate compound, it is difficult to remove impurities, particularly 5'-CMP, which has similar physicochemical properties, from a final product, and in removing 5'-CMP by column chromatography in the related art, the cytidine-5'-diphosphate compound in the solution is diluted, and subsequent concentration is time-consuming and costly. There is also a risk that impurities such as 5'-CMP are generated by decomposition of the cytidine-5'-diphosphate compound due to heating in a concentration step.

Therefore, efficiently separating a cytidine-5'-diphosphate compound and impurities, particularly 5'-CMP, from a solution containing a crude cytidine-5'-diphosphate compound and obtaining a solution containing a cytidine-5'-diphosphate compound at a higher concentration than before purification are important issues in producing a high-purity cytidine-5'-diphosphate compound.

However, in the methods described in Patent Literatures 1 to 3, in order to separate particularly 5'-CMP and a cytidine-5'-diphosphate compound, it is necessary to collect the cytidine-5'-diphosphate compound using a large amount of eluate under moderate elution conditions, and a concentration of the cytidine-5'-diphosphate compound in the eluate decreases, so that a concentration step is necessary for performing crystallization or the like. Particularly, when elution is performed using a volatile acid such as formic acid or hydrochloric acid, in order to remove such an eluent by evaporation, concentration under reduced pressure is mainly performed by heating, and at this time, the heating causes the cytidine-5'-diphosphate compound to decompose and 5'-CMP increases again, which poses a problem as an industrial production method.

The method described in Patent Literature 4 is not suitable as an industrial production method since it is difficult to finally remove an eluent mixed into a fraction containing a cytidine-5'-diphosphate compound. In the method described in Patent Literature 5, it is necessary to wash an inside of a resin column with water after passing a liquid to collect a cytidine-5'-diphosphate compound. Similarly to the methods described in Patent Literatures 1 to 3, a concentration of the cytidine-5'-diphosphate compound decreases compared to before passing through the column, and therefore, application as an industrial production method is limited to a column-passing liquid having a certain concentration or more, and has low versatility.

As described above, a method for separating 5'-CMP from a crude cytidine-5'-diphosphate compound and concentrating the cytidine-5'-diphosphate compound to a higher concentration than before purification has not been reported. Therefore, an object of the present invention is to provide a method for producing a cytidine-5'-diphosphate compound by which 5'-CMP can be efficiently removed from a crude cytidine-5'-diphosphate compound and the cytidine-5'-diphosphate compound can be concentrated to a higher concentration than before purification.

### SOLUTION TO PROBLEM

The present inventors have found that a cytidine-5'-diphosphate compound can be efficiently purified and concentrated by elution using a specific organic acid solution, and have found that particularly, the cytidine-5'-diphosphate compound and 5'-CMP can be efficiently separated, and an eluate containing the cytidine-5'-diphosphate compound at a higher concentration than before purification can be obtained, thereby completing the present invention.

That is, the present invention is as follows.
1. A method for producing a cytidine-5'-diphosphate compound, the method including: the following steps (1) and (2):
   (1) a step of bringing a solution containing a cytidine-5'-diphosphate compound into contact with a basic ion exchange resin to adsorb the cytidine-5'-diphosphate compound onto the basic ion exchange resin, and
   (2) a step of obtaining an eluate by eluting the cytidine-5'-diphosphate compound adsorbed onto the basic ion exchange resin using an aqueous solution containing an organic acid having 2 or more carbon atoms, in which the cytidine-5'-diphosphate compound is a cytidine-5'-diphosphate compound having a cationic substituent.
2. The production method according to above 1, in which a concentration of the cytidine-5'-diphosphate compound contained in the eluate in the step (2) is equal to or higher than a concentration of the cytidine-5'-diphosphate compound in the solution containing a cytidine-5'-diphosphate compound in the step (1).
3. The production method according to above 1 or 2, in which in the step (2), a temperature at which the cytidine-5'-diphosphate compound is eluted from the basic ion exchange resin is 20°C or higher.
4. The production method according to any one of above 1 to 3, in which the organic acid having 2 or more carbon atoms is at least one organic acid selected from acetic acid, propionic acid, and butyric acid.
5. The production method according to any one of above 1 to 4, in which in the step (2), a concentration of the organic acid contained in the aqueous solution is 0.1 mol/L or more.
6. The production method according to any one of above 1 to 5, in which the cytidine-5'-diphosphate compound having a cationic substituent is a compound in which cytidine-5'-diphosphate and a cationic substituent are bonded.
7. The production method according to any one of above 1 to 6, in which the cytidine-5'-diphosphate compound having a cationic substituent is a compound in which a cationic substituent is bonded to a β-phosphate group of cytidine-5'-diphosphate.
8. The production method according to any one of above 1 to 7, in which the organic acid having 2 or more carbon atoms is a carboxylic acid having 2 or more carbon atoms.
9. The production method according to any one of above 1 to 8, in which the cytidine-5'-diphosphate compound having a cationic substituent is at least one selected from cytidine-5'-diphosphate choline and cytidine-5'-diphosphate ethanolamine.

### ADVANTAGEOUS EFFECTS OF INVENTION

According to the production method of the present invention, by providing an elution step using a solution of an organic acid having 2 or more carbon atoms, such as acetic acid or propionic acid, instead of formic acid in the related art, efficient removal of 5'-CMP and obtaining of an eluate containing a cytidine-5'-diphosphate compound at a concentration higher than that before purification can be achieved, and a high-purity cytidine-5'-diphosphate compound can be produced with high efficiency.

### BRIEF DESCRIPTION OF DRAWINGS

[FIG. 1] FIG. 1 is a diagram showing a CDP-choline concentration and a 5'-CMP concentration for each elution fraction in Comparative Example 2, in which RV on a horizontal axis is an abbreviation for resin volume, and represents a volume ratio of an eluate when the resin volume is 1.
[FIG. 2] FIG. 2 is a diagram showing a CDP-choline concentration and a 5'-CMP concentration for each elution fraction in Example 2, in which RV on a horizontal axis is an abbreviation for resin volume, and represents a volume ratio of an eluate when the resin volume is 1.
[FIG. 3] FIG. 3 is a diagram showing a CDP-choline concentration and a 5'-CMP concentration for each elution fraction in Example 4, in which RV on a horizontal axis is an abbreviation for resin volume, and represents a volume ratio of an eluate when the resin volume is 1.

### DESCRIPTION OF EMBODIMENTS

### <Production Method>

A production method of the present invention is characterized by including the following steps (1) and (2).
(1) A step of bringing a solution containing a cytidine-5'-diphosphate compound into contact with a basic ion exchange resin to adsorb the cytidine-5'-diphosphate compound onto the basic ion exchange resin.
(2) A step of obtaining an eluate by eluting the cytidine-5'-diphosphate compound adsorbed onto the basic ion exchange resin using an aqueous solution containing an organic acid having 2 or more carbon atoms.

### (1) A step of bringing a solution containing a cytidine-5'-diphosphate compound into contact with a basic ion exchange resin to adsorb the cytidine-5'-diphosphate compound onto the basic ion exchange resin.

In the present invention, the cytidine-5'-diphosphate compound is a cytidine-5'-diphosphate compound having a cationic substituent. Examples of the cytidine-5'-diphosphate compound having a cationic substituent include a compound in which cytidine-5'-diphosphate and a cationic substituent are bonded. More specifically, the cytidine-5'-diphosphate compound having a cationic substituent is a compound in which a cationic substituent is bonded to a β-phosphate group of cytidine-5'-diphosphate. Here, positions of phosphate groups in cytidine-5'-diphosphate are α-phosphate group and β-phosphate group in order from one closest to cytidine (Chemistry and Education, Vol. 46, No. 6, 1998, pp. 334-337). Examples of the cationic substituent include a substituent having an onium ion such as ammonium, oxonium, phosphonium, sulfonium, iminium, nitrilium, nitrosonium, diazonium, nitronium, or diazenium. Among them, a cationic substituent having an ammonium ion is preferred. Examples of the cytidine-5'-diphosphate compound having a cationic substituent include cytidine-5'-diphosphate choline, and cytidine-5'-diphosphate ethanolamine. Among them, from the viewpoint of purification efficiency, cytidine-5'-diphosphate choline or cytidine-5'-diphosphate ethanolamine is preferred, and cytidine-5'-diphosphate choline is more preferred. One of these may be contained alone in the solution, or two or more thereof may be contained in combination. Modification of the cytidine-5'-diphosphate compound with a functional group is also permissible as long as separation from 5'-CMP can be maintained.

In the step (1) in the production method of the present invention, the solution containing a cytidine-5'-diphosphate compound to be brought into contact with the basic ion exchange resin may be a solution prepared by any method as long as it is a solution containing a cytidine-5'-diphosphate compound. Examples thereof include a solution produced by a chemical synthesis method, a fermentation method, and a method using a biocatalyst.

Examples of the chemical synthesis method for CDP-choline include a method described in K. Kikugawa, M. Ichino, Chem. Pharm. Bull., 19, 1011, 1971. Examples of the chemical synthesis method for CDP-ethanolamine include methods described in H. Ngo, M. F. Dunn et al. Biochemistry, 46, 7713, 2007.; T. Tanaka yakugakuzasshi, 80, 439, 1959.; K. Oertell, C. E. McKenna, M. F. Goodman et al. Biochemistry, 53, 1842, 2014.; T. Tanaka et al. yakugakuzasshi, 85, 863, 1965.; and T. Tanaka et al. yakugakuzasshi, 85, 863, 1965.

Examples of the fermentation method or the method using a biocatalyst include methods described in Japanese Patent No. 3369236; and Y. Liu et al. Appl. Microbiol. Biotechnol., 101, 1409, 2017. The solution containing a cytidine-5'-diphosphate compound used in the step (1) in the production method of the present invention is preferably a solution obtained by removing a solid content from a solution obtained by the fermentation method or the method using a biocatalyst.

As the solution containing a cytidine-5'-diphosphate compound in the step (1) in the production method of the present invention, a solution obtained by treating a solution produced by the chemical synthesis method, the fermentation method, or the method using a biocatalyst with a strongly acidic cation exchange resin and reducing or removing impurities in advance may be used. If necessary, a solution may be used that is treated with activated carbon or decolorized using a non-polar porous synthetic adsorbent such as Diaion HP series (for example, HP20 and HP21) manufactured by Mitsubishi Chemical Corporation, Diaion SP800 series (for example, SP825 and SP850) manufactured by Mitsubishi Chemical Corporation, Diaion SP200 series (for example, SP207) manufactured by Mitsubishi Chemical Corporation, and Amberlight XAD series (for example, XAD4, XAD7HP, XAD16HP, XAD 1180, and XAD2000) manufactured by Rohm and Haas.

The cytidine-5'-diphosphate compound and 5'-CMP can be analyzed by a common method using high-performance liquid chromatography (UV detection).

A concentration of the cytidine-5'-diphosphate compound in the solution containing a cytidine-5'-diphosphate compound in the step (1) is not particularly limited, and is preferably 100 g/L or less, more preferably 85 g/L or less, further preferably 40 g/L or less, even further preferably 15 g/L or less, and most preferably 13 g/L or less, from the viewpoint of purification efficiency. A lower limit of the concentration of the cytidine-5'-diphosphate compound in the solution containing a cytidine-5'-diphosphate compound in the step (1) is not particularly limited, and the concentration is preferably 0.1 g/L or more, more preferably 0.2 g/L or more, further preferably 0.3 g/L or more, and most preferably 0.4 g/L or more.

A ratio of the concentration of 5'-CMP to the concentration of cytidine-5'-diphosphate compound in the solution containing a cytidine-5'-diphosphate compound in step (1) ("5'-CMP concentration (g/L)/concentration of cytidine-5'-diphosphate compound (g/L) × 100 (%)", hereinafter expressed as a "5'-CMP ratio (a ratio % of 5'-CMP to cytidine-5'-diphosphate compound)") is not particularly limited, and the ratio is preferably 150% or less, more preferably 100% or less, further preferably 50% or less, and most preferably 15% or less, from the viewpoint of purification efficiency.

The basic ion exchange resin of the present invention is not particularly limited, and examples thereof include ion exchange resins having primary to tertiary amines or polyamines as exchange groups in a weakly basic anion exchange resin, and an ion exchange resin having quaternary ammonium as an exchange group in a strongly basic anion exchange resin. As the ion exchange resin having quaternary ammonium, either type I having a trimethylammonium group or triethylammonium group or type II having a dimethylethanolammonium group can be suitably used. Examples of a base material of the basic ion exchange resin include a porous type, a macroporous type, a gel type, a styrene type, and an acrylic type.

Specific examples of the basic ion exchange resin include Marathon series (for example, Marathon A, Marathon MSA, and Marathon A2) manufactured by Dow Chemical Company, Monosphere series (for example, Monosphere 77, and Monosphere 550A) manufactured by Dow Chemical Company, 1×2, 1×4, 1×8, 22, 66, and MSA-2 manufactured by Dow Chemical Company, A400, A600, SGA550, A200, A300, A500, A501P, A502PS, A503, A510, A850, A860, A870, SSTA63, SSTA64, PFA520E, A100, A103S, A110, A111S, A133 S, A830W, A845, and A847 manufactured by Purolite, IRA400J, IRA402BL, IRA900J, IRA4580, SCAV4, HPR4002, IRA410J, IRA910CT, HPR4010, HPR4780, IRA67, IRA96SB, and IRA98 manufactured by Organo Corporation, Diaion PA series (for example, PA306S, PA308, PA308L, PA312, PA312L, PA312LOH, PA312LTU, PA312LTUMB, PA316, PA316L, PA318L, PA318LOH, PA408, PA412, PA418, PA418 L, PA418LL, PAF308L, HPA25L, HPA25M, HPA512L, and HPA716) manufactured by Mitsubishi Chemical Corporation (PA is a trademark), Diaion SA series (for example, SA10A, SA10AL, SA10ALLP, SA10AOH, SA10AP, SA10DL, SA11A, SA11AL, SA12A, SA12AL, SA12ALL, SA20A, SA20ALL, SA20ALLP, SA20AP, SA20AP2, SAF11AL, SANUPB, SAT10L, and SAT20L) manufactured by Mitsubishi Chemical Corporation, Diaion UBA series (for example, UBA100, UBA100OH, UBA100OHUP, UBA120, UBA120OH, UBA120OHUP, UBA150, and UBA200) manufactured by Mitsubishi Chemical Corporation, and Diaion WA series (for example, WA10, WA20, WA21J, WA30, WA30C, WA30LL, and WA55) manufactured by Mitsubishi Chemical Corporation.

As the basic ion exchange resin of the present invention, an ion exchange resin having quaternary ammonium as an exchange group in a strongly basic anion exchange resin is more preferred. Specific examples of the ion exchange resin having quaternary ammonium as an exchange group in a strongly basic anion exchange resin include 1×2, 1×4, 1×8, 22, MSA-2, Marathon A, Marathon A2, Marathon MSA, and Monosphere 550A manufactured by Dow Chemical Company, A400, A600, SGA550, A200, A300, A500, A501P, A502PS, A503, A510, A850, A860, A870, SSTA63, SSTA64, and PFA520E manufactured by Purolite, IRA400J, IRA402BL, IRA900J, IRA4580, SCAV4, HPR4002, IRA410J, IRA910CT, and HPR4010 manufactured by Organo Corporation, Diaion PA series (for example, PA306S, PA308, PA308L, PA312, PA312L, PA312LOH, PA312LTU, PA312LTUMB, PA316, PA316L, PA318L, PA318LOH, PA408, PA412, PA418, PA418 L, PA418LL, PAF308L, HPA25L, HPA25M, HPA512L, and HPA716) manufactured by Mitsubishi Chemical Corporation (PA is a trademark), Diaion SA series (for example, SA10A, SA10AL, SA10ALLP, SA10AOH, SA10AP, SA10DL, SA11A, SAI 1AL, SA12A, SA12AL, SA12ALL, SA20A, SA20ALL, SA20ALLP, SA20AP, SA20AP2, SAF11AL, SANUPB, SAT10L, and SAT20L) manufactured by Mitsubishi Chemical Corporation, and Diaion UBA series (for example, UBA100, UBA100OH, UBA100OHUP, UBA120, UBA120OH, UBA120OHUP, UBA150, and UBA200) manufactured by Mitsubishi Chemical Corporation.

An amount of the basic ion exchange resin to be used may be such that a cation amount in the solution containing a cytidine-5'-diphosphate compound, that is, a total molar amount of impurities amino acid, organic acid, chloride ion, and the like contained in the solution in addition to the cytidine-5'-diphosphate compound is within a total exchange capacity of the basic ion exchange resin. As the ionic type of the basic ion exchange resin, either one of a hydroxide ion or an ion in a state of being bonded to an organic acid having 2 or more carbon atoms used for elution, or a combination thereof can be used.

A crosslinking degree of the basic ion exchange resin is not particularly limited as long as it is a crosslinking degree capable of separating the cytidine-5'-diphosphate compound and 5'-CMP, and the crosslinking degree is preferably 1% to 16%, more preferably 2% to 12%, and further preferably 4% to 10%. The basic ion exchange resin may be used in a method in which a solution containing a cytidine-5'-diphosphate compound can be brought into contact with a basic ion exchange resin, and the basic ion exchange resin is preferably used in the present invention in a form of being filled in a column, and any type of column may be used in the present invention.

When the solution containing a cytidine-5'-diphosphate compound is brought into contact with the basic ion exchange resin by passing the solution through a column filled with the resin to adsorb the cytidine-5'-diphosphate compound onto the resin, for example, a column-passing rate when passing through a column filled with a basic ion exchange resin with a crosslinking degree of 1% to 16% is a space velocity (a volume ratio (/hour) of the solution passed through the column per hour when the resin volume of the ion exchange resin is 1, hereinafter referred to as "SV"), and is preferably SV = 0.1 to 5, more preferably SV = 0.2 to 4, and further preferably SV = 0.4 to 3.

### (2) A step of obtaining an eluate by eluting the cytidine-5'-diphosphate compound adsorbed onto the basic ion exchange resin using an aqueous solution containing an organic acid having 2 or more carbon atoms

The step (2) is a step of obtaining an eluate by passing an aqueous solution containing an organic acid having 2 or more carbon atoms through the column with the basic ion exchange resin onto which the cytidine-5'-diphosphate compound is adsorbed in the step (1), thereby eluting the cytidine-5'-diphosphate compound from the resin to separate and purify the cytidine-5'-diphosphate compound.

The organic acid having 2 or more carbon atoms is preferably a carboxylic acid having 2 or more carbon atoms. Examples of the organic acid having 2 or more carbon atoms include acetic acid, propionic acid, butyric acid, valeric acid, lactic acid, glycolic acid, pyruvic acid, gluconic acid, tartaric acid, oxalic acid, malonic acid, succinic acid, glutaric acid, adipic acid, malic acid, fumaric acid, maleic acid, citric acid, isocitric acid, and ascorbic acid. Among them, acetic acid, propionic acid, and butyric acid are preferred, and acetic acid and propionic acid are more preferred. One of the organic acid having 2 or more carbon atoms may be used alone or a plurality thereof may be used in combination. An upper limit of the number of carbon atoms in the organic acid is not particularly limited, and from the viewpoint of solubility in water, the number of carbon atoms is preferably 8 or less, more preferably 6 or less, and further preferably 3 or less. The organic acid having 2 or more carbon atoms may be a salt of an organic acid having 2 or more carbon atoms.

A concentration of the organic acid having 2 or more carbon atoms in the aqueous solution containing an organic acid having 2 or more carbon atoms is preferably 0.1 mol/L or more, and more preferably 0.35 mol/L or more, from the viewpoint of increasing the purification efficiency of the cytidine-5'-diphosphate compound. An upper limit of the concentration of the organic acid having 2 or more carbon atoms in the aqueous solution is not particularly limited, and the concentration is preferably 2.0 mol/L or less, more preferably 1.0 mol/L or less, further preferably 0.6 mol/L or less, and most preferably 0.45 mol/L or less, from the viewpoint of a balance between the amount of the organic acid to be used and the effect.

The eluate in the step (2) (the eluate containing a cytidine-5'-diphosphate compound, hereinafter also referred to as a cytidine-5'-diphosphate compound-containing eluate) can be obtained by collecting a liquid obtained by passing the aqueous solution containing an organic acid having 2 or more carbon atoms through the column with the basic ion exchange resin in two or more fractions in order to separate impurities and the cytidine-5'-diphosphate compound and also to make it possible to concentrate the cytidine-S'-diphosphate compound as necessary. The obtained two or more fractions containing the cytidine-5'-diphosphate compound may be mixed to form a cytidine-5'-diphosphate compound-containing eluate. The liquid can be collected in more preferably three or more fractions, and further preferably in four or more fractions. Before the step of mixing the fractions containing the cytidine-5'-diphosphate compound, a step of analyzing the cytidine-5'-diphosphate compound and the impurities such as 5'-CMP, which are contained in each fraction, by HPLC may be included. The cytidine-5'-diphosphate compound and the impurities such as 5'-CMP can be analyzed by, for example, a common method using high-performance liquid chromatography (UV detection).

A concentration of the cytidine-5'-diphosphate compound contained in the eluate in the step (2) is preferably equal to or higher than the concentration of the cytidine-5'-diphosphate compound in the solution containing a cytidine-5'-diphosphate compound in the step (1). Specifically, for example, a concentration ratio, that is, concentration (g/L) of cytidine-5'-diphosphate compound contained in eluate in step (2)/concentration (g/L) of cytidine-5'-diphosphate compound in solution to be brought into contact with basic ion exchange resin in step (1) is preferably 1.0 times or more, and more preferably 1.1 times or more. An upper limit of the concentration ratio is not particularly limited, and the concentration ratio is, for example, 160 times or less.

A 5'-CMP ratio (a ratio % of 5'-CMP to cytidine-5'-diphosphate compound) of the eluate in the step (2) is preferably 1.00% or less, more preferably 0.20% or less, and further preferably 0.10% or less. A lower limit of the ratio of 5'-CMP to cytidine-5'-diphosphate compound is not particularly limited, and the ratio is, for example, 0% or more.

A collection ratio (%) of the cytidine-5'-diphosphate compound in the step (2), that is, mass (g) of cytidine-5'-diphosphate compound contained in eluate in step (2)/mass (g) of cytidine-5'-diphosphate compound contained in solution containing cytidine-5'-diphosphate compound in step (1) × 100 (%) is preferably 60% or more, more preferably 80% or more, and further preferably 89% or more. An upper limit of the collection ratio is not particularly limited, and the collection ratio is, for example, less than 100%.

In the step (2), a temperature at which the cytidine-5'-diphosphate compound is eluted from the basic ion exchange resin is preferably 20°C or higher, more preferably 30°C or higher, and further preferably 40°C or higher, from the viewpoint of enhancing separability between the cytidine-5'-diphosphate compound and 5'-CMP during elution. An upper limit of the elution temperature is not particularly limited, and the elution temperature is preferably 60°C or lower from the viewpoint of preventing formation of 5'-CMP due to thermal decomposition of the cytidine-5'-diphosphate compound, In the present invention, the elution temperature refers to a temperature of a column and/or a temperature of the aqueous solution containing an organic acid having 2 or more carbon atoms.

From the viewpoint of enhancing the separability between the cytidine-5'-diphosphate compound and 5'-CMP, when the organic acid used for elution is an organic acid having 2 carbon atoms (for example, acetic acid), the elution temperature is preferably 30°C to 60°C, and more preferably 40°C to 60°C. On the other hand, when the organic acid used for elution is an organic acid having 3 or more carbon atoms (for example, propionic acid), the elution temperature is preferably 20°C to 60°C, more preferably 30°C to 60°C, and further preferably 40°C to 60°C.

It is known that when a temperature is changed in an ion exchange reaction, a retention time of a compound changes due to changes in separation equilibrium, diffusion rate, degree of dissociation, viscosity of the eluent, and the like. Although the influence of the temperature on the ion exchange reaction varies depending on the compound and also varies depending on a carrier and an eluent, the present inventors have found that the separability between the cytidine-5'-diphosphate compound and 5'-CMP tends to be improved by increasing the elution temperature. Further, the present inventors have confirmed that hydrolysis of the cytidine-5'-diphosphate compound by heating proceeds even at 30°C, and under a condition of 40°C or higher, decomposition progresses significantly, with more than 10 times as much 5'-CMP being produced as a by-product compared to a condition of 20°C, but the present inventors have found that the effect of improving the separability between the cytidine-5'-diphosphate compound and 5'-CMP by increasing the elution temperature is greater than disadvantages of decomposition by heating.

In the step (2), a column-passing rate at which the aqueous solution containing an organic acid having 2 or more carbon atoms is passed through the column with the basic ion exchange resin is preferably SV = 0.1 to 2, more preferably SV = 0.2 to 1.5, and further preferably SV = 0.4 to 1.

Through the above steps (1) and (2), the cytidine-5'-diphosphate compound and the impurities contained in the solution, such as 5'-CMP, UMP, UDP, UTP, UDP-choline, UDP-ethanolamine, CDP, CTP, inorganic phosphoric acid, and polyphosphoric acid can be efficiently separated, and a high-purity cytidine-5'-diphosphate compound can be produced. Particularly, the above steps (1) and (2) allow efficient separation of the cytidine-5'-diphosphate compound and 5'-CMP.

The cytidine-5'-diphosphate compound-containing eluate obtained by the elution step may be treated with activated carbon or, if necessary, decolorized using a non-polar porous synthetic adsorbent such as Diaion HP series (for example, HP20 and HP21) manufactured by Mitsubishi Chemical Corporation, Diaion SP800 series (for example, SP825 and SP850) manufactured by Mitsubishi Chemical Corporation, Diaion SP200 series (for example, SP207) manufactured by Mitsubishi Chemical Corporation, Amberlight XAD series (for example, XAD4, XAD7HP, XAD16HP, XAD1180, and XAD2000) manufactured by Rohm and Haas.

Further, in order to obtain a free-form crystal of the cytidine-5'-diphosphate compound, a cytidine-5'-diphosphate compound crystal can be obtained by adjusting the pH of the eluate containing the cytidine-5'-diphosphate compound or the liquid after the decolorization treatment to preferably 2.0 to 4.0 with an acid or an alkali as necessary, performing concentration as necessary, then adjusting the concentration of the cytidine-5'-diphosphate compound to preferably 50 g/L to 800 g/L, more preferably 100 g/L to 700 g/L, and using an organic solvent, preferably a hydrophilic organic solvent such as acetone, ethanol, methanol, or propanol.

In order to obtain a salt crystal of the cytidine-5'-diphosphate compound, for example, a cytidine-5'-diphosphate compound sodium salt crystal can be obtained by adjusting the pH of the eluate containing the cytidine-5'-diphosphate compound to preferably 5.0 to 9.5 with sodium hydroxide, performing a decolorization treatment and a concentration treatment as necessary, then adjusting the concentration of the cytidine-5'-diphosphate compound to preferably 50 g/L to 800 g/L and more preferably 100 g/L to 700 g/L, and using an organic solvent, preferably a hydrophilic organic solvent such as acetone, ethanol, methanol, or propanol.

Examples of the method for obtaining a cytidine-5'-diphosphate compound crystal using an organic solvent include a method in which an organic solvent is added to a cytidine-5'-diphosphate compound solution to precipitate a crystal, and a method in which a cytidine-5'-diphosphate compound solution is added dropwise to a large amount of an organic solvent to precipitate a crystal.

Hereinafter, the present invention will be further specifically described with reference to Examples, but the present invention is not limited to these Examples.

### EXAMPLE

### [Analysis Example 1]

Quantitative analysis of CDP-choline, 5'-CMP, and CDP-ethanolamine by HPLC was performed under the following conditions.
Device to be used: system controller (CBM-20A), detector (SPD-20A), pump (LC-20AT), autosampler (SIL-20ACXR), column oven (CTO-10ASvp), data analysis software (LabSolutions Version 5.85) (all manufactured by Shimadzu Corporation)
Detector: ultraviolet ray absorption photometer (measured wavelength 254 nm)
Column: Shodex Asahipak NH2P-50 4E 4.6 × 250 mm (manufactured by Showa Denko)
Mobile phase: 0.03 mol/L potassium dihydrogen phosphate aqueous solution adjusted to pH 3.5 with phosphoric acid (20.41 g of potassium dihydrogen phosphate was dissolved in 5 L of distilled water, and the pH was adjusted to pH 3.5 by adding phosphoric acid).
Column temperature: 40°C
Flow rate: 0.50 ml/min
Sample injection amount: 10 µL
Retention time: CDP-choline 7.3 min, 5'-CMP 16.0 min, CDP-ethanolamine 9.1 min

Each elution fraction in the step (2) was diluted 100 times or adjusted to be close to a concentration of a standard sample, and used for analysis. Concentrations of CDP-choline, 5'-CMP, and CDP-ethanolamine contained in each elution fraction were determined, using 0.02 g/L of CDP-choline, 0.01 g/L of 5'-CMP, and 0.018 g/L of CDP-ethanolamine as standard samples, respectively, from peak area values of the standard samples by a one-point calibration curve method.

For example, for CDP-choline, the concentration can be calculated as follows: (CDP-choline concentration (g/L) of elution fraction) = 0.02 × B/A × x, where A is a peak area value of a CDP-choline standard and B is a peak area value of an elution fraction diluted x times.

### [Analysis Example 2]

Quantitative analysis of a CDP trisodium salt by HPLC was performed under the following conditions.
Device to be used: system controller (CBM-20A), detector (SPD-20A), pump (LC-20AD), autosampler (SIL-20AC), column oven (CTO-20AC), data analysis software (LabSolutions Version 5.71 SP1) (all manufactured by Shimadzu Corporation)
Detector: ultraviolet ray absorption photometer (measured wavelength 254 nm)
Column: Two Partisil 10 SAX 4.0 × 250 mm in series connection (Hichrom)
Mobile phase: 0.06 mol/L potassium dihydrogen phosphate aqueous solution adjusted to pH 3.5 with phosphoric acid (40.83 g of potassium dihydrogen phosphate was dissolved in 5 L of distilled water, and the pH was adjusted to pH 3.5 by adding phosphoric acid).
Column temperature: 30°C
Flow rate: 0.45 ml/min
Sample injection amount: 10 µL
Retention time: CDP trisodium salt 82.5 min

### [Comparative Example 1]

With reference to Indian Patent Application Publication No. 2012MUM02534, experiments were carried out using a 0.10 mol/L formic acid aqueous solution. As a solution containing a cytidine-5'-diphosphate compound, 210 mL of an aqueous solution containing CDP-choline (manufactured by Kyowa Hakko Bio Co., Ltd.) at a concentration of 7.02 g/L and 5'-CMP (manufactured by Tokyo Chemical Industry Co., Ltd.) at a concentration of 0.37 g/L [a ratio of 5'-CMP concentration to CDP-choline concentration in the solution (hereinafter referred to as a "ratio of 5'-CMP to CDP-choline") is 5.3%] was prepared, and the aqueous solution was passed through a column with 150 mL of OH⁻ type Marathon A (manufactured by Dow Chemical Company) at SV = 1.25 for adsorption.

Next, a temperature outside the column was adjusted to 20°C, and 225 mL of water was passed through the column at SV = 1.10. Finally, while the column was kept at 20°C, 3 L of 0.10 mol/L formic acid aqueous solution (a diluted special grade reagent manufactured by FUJIFILM Wako Pure Chemical Corporation) was passed through the column at SV = 0.85, and 29.9 mL each of eluate was obtained. The fractions containing CDP-choline were mixed, and as a result, 239 mL of a solution having a CDP-choline concentration of 6.08 g/L was obtained. A 5'-CMP concentration of the solution was 0.40 g/L (a ratio of 5'-CMP to CDP-choline 6.6%). A collection ratio of CDP-choline was 98.6%.

### [Comparative Example 2]

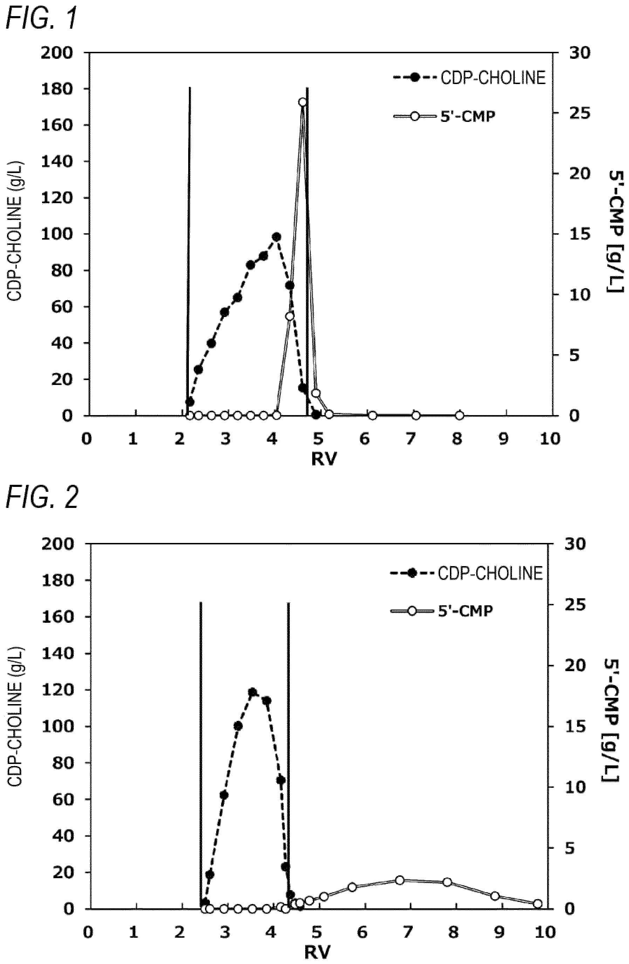

As a solution containing a cytidine-5'-diphosphate compound, 3.20 L of an aqueous solution containing CDP-choline at a concentration of 7.11 g/L and 5'-CMP at a concentration of 0.36 g/L (a ratio of 5'-CMP to CDP-choline 5.1%) was prepared, and the aqueous solution was passed through a column with 150 mL of OH⁻ type Marathon MSA (manufactured by Dow Chemical Company) at SV = 1.25 for adsorption. Next, a temperature outside the column was adjusted to 20°C, and 225 mL of water was passed through the column at SV = 1.10. Finally, while the column was kept at 20°C, 1350 ml of 0.35 mol/L formic acid aqueous solution was passed through the column at SV = 0.85, and 14.1 mL each of eluate was obtained. FIG. 1 shows a result of quantifying a CDP-choline concentration and a 5'-CMP concentration by HPLC for each elution fraction.

The fractions containing CDP-choline were mixed, and as a result, 381 mL of a solution having a CDP-choline concentration of 59.7 g/L was obtained. A 5'-CMP concentration of the solution was 2.7 g/L (a ratio of 5'-CMP to CDP-choline 4.5%). A collection ratio of CDP-choline was 100%.

### [Example 1]

As a solution containing a cytidine-5'-diphosphate compound, 3.20 L of an aqueous solution containing CDP-choline at a concentration of 7.11 g/L and 5'-CMP at a concentration of 0.36 g/L (a ratio of 5'-CMP to CDP-choline 5.1%) was prepared, and the aqueous solution was passed through a column with 150 mL of OH⁻ type Marathon MSA (manufactured by Dow Chemical Company) at SV = 1.25 for adsorption. Next, a temperature outside the column was adjusted to 20°C, and 225 mL of water was passed through the column at SV = 1.10. Finally, while the column was kept at 20°C, 1350 mL of 0.35 mol/L acetic acid aqueous solution (a diluted special grade reagent manufactured by FUJIFILM Wako Pure Chemical Corporation) was passed through the column at SV = 0.85, and 14.6 mL each of eluate was obtained. The fractions containing CDP-choline were mixed, and as a result, 351 mL of a solution having a CDP-choline concentration of 65.3 g/L was obtained. A 5'-CMP concentration of the solution was 0.10 g/L (a ratio of 5'-CMP to CDP-choline 0.15%). A collection ratio of CDP-choline was 100%.

### [Example 2]

As a solution containing a cytidine-5'-diphosphate compound, 3.21 L of an aqueous solution containing CDP-choline at a concentration of 7.13 g/L and 5'-CMP at a concentration of 0.38 g/L (a ratio of 5'-CMP to CDP-choline 5.3%) was prepared, and the aqueous solution was passed through a column with 150 mL of OH⁻ type Marathon MSA (manufactured by Dow Chemical Company) at SV = 1.25 for adsorption. Next, a temperature outside the column was adjusted to 20°C, and 225 mL of water was passed through the column at SV = 1.10. Finally, while the column was kept at 20°C, 1350 mL of 0.35 mol/L propionic acid aqueous solution (a diluted Wako special grade reagent manufactured by FUJIFILM Wako Pure Chemical Corporation) was passed through the column at SV = 0.85, and 15.6 mL each of eluate was obtained. FIG. 2 shows a result of quantifying a CDP-choline concentration and a 5'-CMP concentration by HPLC for each elution fraction.

The fractions containing CDP-choline were mixed, and as a result, 281 mL of a solution having a CDP-choline concentration of 78.2 g/L was obtained. A 5'-CMP concentration of the solution was 0.03 g/L (a ratio of 5'-CMP to CDP-choline 0.04%). A collection ratio of CDP-choline was 96.0%.

### [Example 3]

As a solution containing a cytidine-5'-diphosphate compound, 3.64 L of an aqueous solution containing CDP-choline at a concentration of 7.12 g/L and 5'-CMP at a concentration of 0.41 g/L (a ratio of 5'-CMP to CDP-choline 5.8%) was prepared, and the aqueous solution was passed through a column with 150 mL of OH⁻ type Marathon A (manufactured by Dow Chemical Company) at SV = 1.25 for adsorption. Next, a temperature outside the column was adjusted to 20°C, and 225 mL of water was passed through the column at SV = 1.10. Finally, while the column was kept at 20°C, 1350 mL of 0.45 mol/L acetic acid aqueous solution was passed through the column at SV = 0.85, and 14.8 mL each of eluate was obtained. The fractions containing CDP-choline were mixed, and as a result, 415 mL of a solution having a CDP-choline concentration of 63.2 g/L was obtained. A 5'-CMP concentration of the solution was 0.32 g/L (a ratio of 5'-CMP to CDP-choline 0.51%). A collection ratio of CDP-choline was 100%.

### [Example 4]

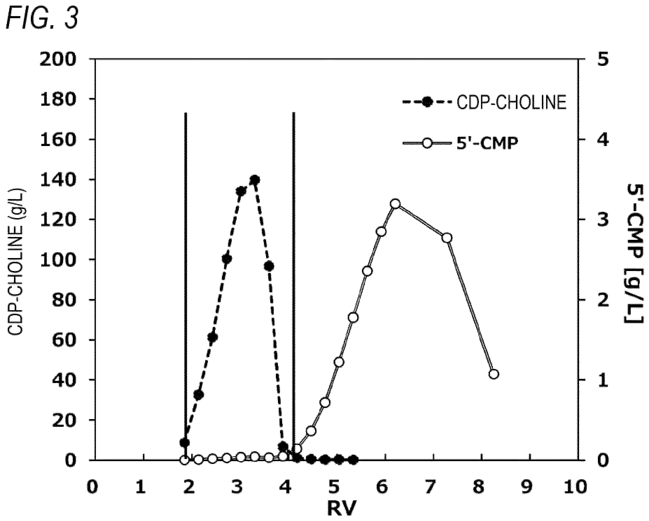

As a solution containing a cytidine-5'-diphosphate compound, 3.64 L of an aqueous solution containing CDP-choline at a concentration of 7.12 g/L and 5'-CMP at a concentration of 0.41 g/L (a ratio of 5'-CMP to CDP-choline 5.8%) was prepared, and the aqueous solution was passed through a column with 150 mL of OH⁻ type Marathon A (manufactured by Dow Chemical Company) at SV = 1.25 for adsorption. Next, a temperature outside the column was adjusted to 30°C, and 225 mL of water was passed through the column at SV = 1.10. Finally, while the column was kept at 30°C, 1350 mL of 0.45 mol/L acetic acid aqueous solution was passed through the column at SV = 0.85, and 14.6 mL each of eluate was obtained. FIG. 3 shows a result of quantifying a CDP-choline concentration and a 5'-CMP concentration by HPLC for each elution fraction. The fractions containing CDP-choline were mixed, and as a result, 349 mL of a solution having a CDP-choline concentration of 71.4 g/L was obtained. A 5'-CMP concentration of the solution was 0.03 g/L (a ratio of 5'-CMP to CDP-choline 0.04%). A collection ratio of CDP-choline was 96.1%.

### [Example 5]

As a solution containing a cytidine-5'-diphosphate compound, 3.64 L of an aqueous solution containing CDP-choline at a concentration of 7.13 g/L and 5'-CMP at a concentration of 0.38 g/L (a ratio of 5'-CMP to CDP-choline 5.3%) was prepared, and the aqueous solution was passed through a column with 150 mL of OH⁻ type Marathon A (manufactured by Dow Chemical Company) at SV = 1.25 for adsorption. Next, a temperature outside the column was heated to 60°C, and 225 mL of water was passed through the column at SV = 1.10. Finally, while the column was kept at 60°C, 1350 mL of 0.45 mol/L acetic acid aqueous solution was passed through the column at SV = 0.85, and 14.8 mL each of eluate was obtained. The fractions containing CDP-choline were mixed, and as a result, 354 mL of a solution having a CDP-choline concentration of 71.2 g/L was obtained. A 5'-CMP concentration of the solution was 0.01 g/L (a ratio of 5'-CMP to CDP-choline 0.01%). A collection ratio of CDP-choline was 97.1%.

### [Comparative Example 3]

As a solution containing a cytidine-5'-diphosphate compound, 3.20 L of an aqueous solution containing CDP-choline at a concentration of 7.10 g/L and 5'-CMP at a concentration of 0.38 g/L (a ratio of 5'-CMP to CDP-choline 5.4%) was prepared, and the aqueous solution was passed through a column with 150 mL of OH⁻ type Marathon MSA (manufactured by Dow Chemical Company) at SV = 1.25 for adsorption. Next, a temperature outside the column was heated to 40°C, and 225 mL of water was passed through the column at SV = 1.10. Finally, while the column was kept at 40°C, 1350 mL of 0.45 mol/L formic acid aqueous solution was passed through the column at SV = 0.85, and 14.7 mL each of eluate was obtained. The fractions containing CDP-choline were mixed, and as a result, 323 mL of a solution having a CDP-choline concentration of 67.4 g/L was obtained. A 5'-CMP concentration of the solution was 3.5 g/L (a ratio of 5'-CMP to CDP-choline 5.2%). A collection ratio of CDP-choline was 95.8%.

### [Example 6]

As a solution containing a cytidine-5'-diphosphate compound, 3.20 L of an aqueous solution containing CDP-choline at a concentration of 7.10 g/L and 5'-CMP at a concentration of 0.38 g/L (a ratio of 5'-CMP to CDP-choline 5.4%) was prepared, and the aqueous solution was passed through a column with 150 mL of OH⁻ type Marathon MSA (manufactured by Dow Chemical Company) at SV = 1.25 for adsorption. Next, a temperature outside the column was heated to 40°C, and 225 mL of water was passed through the column at SV = 1.10. Finally, while the column was kept at 40°C, 1350 ml of 0.45 mol/L acetic acid aqueous solution was passed through the column at SV = 0.85, and 15.2 mL each of eluate was obtained. The fractions containing CDP-choline were mixed, and as a result, 305 mL of a solution having a CDP-choline concentration of 70.8 g/L was obtained. A 5'-CMP concentration of the solution was 0.05 g/L (a ratio of 5'-CMP to CDP-choline 0.07%). A collection ratio of CDP-choline was 95.0%.

### [Example 7]

As a solution containing a cytidine-5'-diphosphate compound, 2.90 L of an aqueous solution containing CDP-choline at a concentration of 7.05 g/L and 5'-CMP at a concentration of 0.37 g/L (a ratio of 5'-CMP to CDP-choline 5.2%) was prepared, and the aqueous solution was passed through a column with 150 mL of OH⁻ type Marathon A (manufactured by Dow Chemical Company) at SV = 1.25 for adsorption. Next, a temperature outside the column was heated to 40°C, and 225 mL of water was passed through the column at SV = 1.10. Finally, while the column was kept at 40°C, 3 L of 0.10 mol/L acetic acid aqueous solution was passed through the column at SV = 0.85, and 26.8 mL each of eluate was obtained. The fractions containing CDP-choline were mixed, and as a result, 885 mL of a solution having a CDP-choline concentration of 22.6 g/L was obtained. A 5'-CMP concentration of the solution was 0.003 g/L (a ratio of 5'-CMP to CDP-choline 0.01%). A collection ratio of CDP-choline was 97.8%.

### [Example 8]

As a solution containing a cytidine-5'-diphosphate compound, 2.15 L of an aqueous solution containing CDP-choline at a concentration of 7.24 g/L and 5'-CMP at a concentration of 0.38 g/L (a ratio of 5'-CMP to CDP-choline 5.2%) was prepared, and the aqueous solution was passed through a column with 139 mL of OH⁻ type PFA520E (manufactured by Purolite) at SV = 1.25 for adsorption. Next, a temperature outside the column was heated to 40°C, and 209 mL of water was passed through the column at SV = 1.10. Finally, while the column was kept at 40°C, 1500 mL of 0.10 mol/L acetic acid aqueous solution was passed through the column at SV = 0.85, and 13.9 mL each of eluate was obtained. The fractions containing CDP-choline were mixed, and as a result, 487 mL of a solution having a CDP-choline concentration of 31.0 g/L was obtained. A 5'-CMP concentration of the solution was 0.005 g/L (a ratio of 5'-CMP to CDP-choline 0.02%). A collection ratio of CDP-choline was 97.0%.

### [Example 9]

As a solution containing a cytidine-5'-diphosphate compound, 1.00 L of an aqueous solution containing CDP-choline at a concentration of 7.04 g/L and 5'-CMP at a concentration of 0.37 g/L (a ratio of 5'-CMP to CDP-choline 5.3%) was prepared, and the aqueous solution was passed through a column with 100 mL of OH⁻ type Monosphere 77 (manufactured by Dow Chemical Company) at SV = 1.25 for adsorption. Next, a temperature outside the column was heated to 40°C, and 150 mL of water was passed through the column at SV = 1.10. Finally, while the column was kept at 40°C, 1500 mL of 0.40 mol/L acetic acid aqueous solution was passed through the column at SV = 0.85, and 15.0 mL each of eluate was obtained. The fractions containing CDP-choline were mixed, and as a result, 285 mL of a solution having a CDP-choline concentration of 24.4 g/L was obtained. A 5'-CMP concentration of the solution was 0.005 g/L (a ratio of 5'-CMP to CDP-choline 0.02%). A collection ratio of CDP-choline was 98.8%.

Table 1 shows the above results.

**[Table 1]**

| | Solution in step (1) | | Anion exchange resin treatment condition | | | Eluate in step (2) | | | | | |
|---|---|---|---|---|---|---|---|---|---|---|---|
| | CDP-choline concentration (g/L) | 5'-CMP ratio (ratio % of 5'-CMP to CDP-choline) | Resin type | Resin product name | Resin amount (mL) | Elution temperature (°C) | Organic acid used for elution | Concentration (mol/L) of organic acid used for elution | CDP-choline concentration (g/L) | 5'-CMP ratio (ratio % of 5'-CMP to CDP-choline) | Collection ratio (%) |
| Comparative Example 1 | 7.02 | 5.3 | Strongly basic type I | Marathon A | 150 | 20 | Formic acid | 0.10 | 6.08 | 6.6 | 98.6 |
| Comparative Example 2 | 7.11 | 5.1 | Strongly basic type I | Marathon MSA | 150 | 20 | Formic acid | 0.35 | 59.7 | 4.5 | 100 |
| Example 1 | 7.11 | 5.1 | Strongly basic type I | Marathon MSA | 150 | 20 | Acetic acid | 0.35 | 65.3 | 0.15 | 100 |
| Example 2 | 7.13 | 5.3 | Strongly basic type I | Marathon MSA | 150 | 20 | Propionic acid | 0.35 | 78.2 | 0.04 | 96.0 |
| Example 3 | 7.12 | 5.8 | Strongly basic type I | Marathon A | 150 | 20 | Acetic acid | 0.45 | 63.2 | 0.51 | 100 |
| Example 4 | 7.12 | 5.8 | Strongly basic type I | Marathon A | 150 | 30 | Acetic acid | 0.45 | 71.4 | 0.04 | 96.1 |
| Example 5 | 7.13 | 5.3 | Strongly basic type I | Marathon A | 150 | 60 | Acetic acid | 0.45 | 71.2 | 0.01 | 97.1 |
| Comparative Example 3 | 7.10 | 5.4 | Strongly basic type I | Marathon MSA | 150 | 40 | Formic acid | 0.45 | 67.4 | 5.2 | 95.8 |
| Example 6 | 7.10 | 5.4 | Strongly basic type I | Marathon MSA | 150 | 40 | Acetic acid | 0.45 | 70.8 | 0.07 | 95.0 |
| Example 7 | 7.05 | 5.2 | Strongly basic type I | Marathon A | 150 | 40 | Acetic acid | 0.10 | 22.6 | 0.01 | 97.8 |
| Example 8 | 7.24 | 5.2 | Strongly basic type I | PFA520E | 139 | 40 | Acetic acid | 0.10 | 31.0 | 0.02 | 97.0 |
| Example 9 | 7.04 | 5.3 | Weakly basic | Monosphere 77 | 100 | 40 | Acetic acid | 0.40 | 24.4 | 0.02 | 98.8 |

As shown in Table 1, it was found that in Examples 1 to 9 in which the eluate was obtained by bringing the solution containing a cytidine-5'-diphosphate compound into contact with the basic ion exchange resin to adsorb the cytidine-5'-diphosphate compound onto the basic ion exchange resin, and then eluting the cytidine-5'-diphosphate compound adsorbed onto the basic ion exchange resin using the aqueous solution containing an organic acid having two or more carbon atoms, the high-purity cytidine-5'-diphosphate compound was obtained with high efficiency as compared with Comparative Examples 1 to 3 in which the cytidine-5'-diphosphate compound was eluted using the aqueous solution containing an organic acid having one carbon atom.

### [Example 10]

Organic chemical synthesis of CDP-choline was performed according to a known method (K. Kikugawa, M. Ichino, Chem. Pharm. Bull., 19, 1011, 1971.). To 20 mL of water, 6.6 g of phosphocholine chloride calcium salt tetrahydrate (manufactured by Tokyo Chemical Industry Co., Ltd.) was added, followed by stirring at 80°C until dissolution. To the solution, 2.52 g of oxalic acid dihydrate (a special grade reagent manufactured by FUJIFILM Wako Pure Chemical Corporation) was added, followed by stirring for 10 minutes.

The resulting white precipitate was collected by filtration, and the resulting filtrate was concentrated at 50°C under reduced pressure. Further, 16 mL of DMF (manufactured by FUJIFILM Wako Pure Chemical Corporation, for organic synthesis) was added thereto, followed by concentrating at 70°C under reduced pressure. After cooling to 0°C, a solution obtained by dissolving 4.4 g of p-toluenesulfonyl chloride (manufactured by Nacalai Tesque, Inc.) in 6 mL of DMF was added dropwise thereto, followed by stirring until uniform. To this, 2.0 g of cytidine-5'-monophosphate was added, followed by stirring overnight at room temperature. After cooling to 0°C again, 250 mL of water was added thereto, and the pH was adjusted to 3.5 by using 28 mass% ammonia water (a special grade reagent manufactured by FUJIFILM Wako Pure Chemical Corporation). Two of the above operations were simultaneously performed, and after mixing both, water was added to obtain 5050 mL of a solution containing a cytidine-5'-diphosphate compound. At this time, the solution containing a cytidine-5'-diphosphate compound contained CDP-choline at a concentration of 0.486 g/L and 5'-CMP at a concentration of 0.0717 g/L (a ratio of 5'-CMP to CDP-choline 14.8%).

2450 mL of the solution containing a cytidine-5'-diphosphate compound thus obtained was passed through a column with 150 mL of OH⁻ type PA412 (manufactured by Mitsubishi Chemical Corporation) at SV = 1.25 for adsorption. Thereafter, a temperature outside the column was heated to 60°C, and 225 mL of water was passed through the column at SV = 1.10. Finally, while the column was kept at 60°C, 1350 mL of 0.50 mol/L propionic acid aqueous solution was passed through the column at SV = 0.85, and 15.2 mL each of eluate was obtained. The fractions containing CDP-choline were mixed, and as a result, 106 mL of a solution having a CDP-choline concentration of 10.7 g/L was obtained. A 5'-CMP concentration of the solution was 0.003 g/L (a ratio of 5'-CMP to CDP-choline 0.03%). A collection ratio of CDP-choline was 95.3%. From the results, it is found that the production method of the present invention can also be applied to a solution containing a cytidine-5'-diphosphate compound obtained by an organic synthesis method.

### [Example 11]

A culture solution having a CDP-choline concentration of 30.8 g/L was obtained by a method described in Japanese Patent No. 3369236, and the pH was adjusted to 3.0 with sulfuric acid. Next, bacterial cells were separated by centrifugation (8000 rpm, 10 minutes), and water was added to the obtained supernatant to obtain 10.9 L of an aqueous solution containing CDP-choline at a concentration of 12.2 g/L as a solution containing a cytidine-5'-diphosphate compound.

The solution containing a cytidine-5'-diphosphate compound was passed through a column with 8.4 L of H⁺ type strongly acidic cation exchange resin at SV = 0.26 for adsorption while an external temperature of the column was maintained at 10°C. Subsequently, cold water at 10°C was passed through the column, and when elution of CDP-choline started, the solution was continuously passed through a column with 900 mL of OH⁻ type PA412 for adsorption. Thereafter, a temperature outside the PA412 column was heated to 40°C, and 2.7 L of water was passed through the column at SV = 1.10. Finally, while the column was kept at 40°C, 5.4 L of 0.47 mol/L acetic acid aqueous solution was passed through the column at SV = 0.85, and 61 mL each of eluate was obtained.

The fractions containing CDP-choline were mixed, and as a result, 2.85 L of a solution having a CDP-choline concentration of 42.8 g/L was obtained. A 5'-CMP concentration of the solution was 0.01 g/L or less (a ratio of 5'-CMP to CDP-choline 0.02% or less). A collection ratio of CDP-choline was 91.7%. From the results, it is found that the production method of the present invention can also be applied to a solution containing a cytidine-5'-diphosphate compound obtained by a culture method.

### [Example 12]

A reaction formula in organic synthesis of CDP-ethanolamine is shown below. In the reaction formula below, "rt" refers to room temperature.

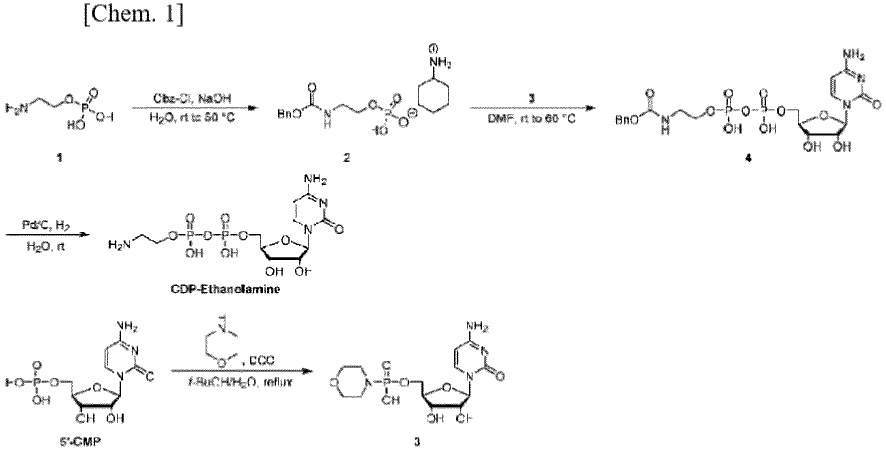

Synthesis of the compound 2 was performed with reference to a known literature (H. Ngo, M. F. Dunn et al. Biochemistry, 46, 7713, 2007.; T. Tanaka yakugakuzasshi, 80, 439, 1959.). To 18.9 mL of water, 6.80 g of sodium hydroxide (a special grade reagent manufactured by FUJIFILM Wako Pure Chemical Corporation) was added, followed by stirring until completely dissolved. After the solution was cooled to 0°C, 8.00 g of phosphoethanolamine (manufactured by Tokyo Chemical Industry Co., Ltd.) was added, followed by stirring until dissolved. To this, 7.99 mL of Z-chloride (manufactured by Tokyo Chemical Industry Co., Ltd.) was added dropwise, followed by stirring at room temperature for 2 hours. The mixture was further stirred at 50°C for 1 hour, and then stirred overnight at room temperature. A liquid prepared by adding 1 L of water thereto was passed through a column with 850 mL of H⁺ type strongly acidic cation exchange resin. After passing through the column, washing with water was performed to obtain a total of 5 L of eluate. The liquid was concentrated under reduced pressure to obtain 500 mL of crude product liquid.

The crude product liquid was neutralized with cyclohexylamine (Wako special grade manufactured by FUJIFILM Wako Pure Chemical Corporation) until the pH reached 4.5, and then the solvent was completely distilled off under reduced pressure. To the precipitated white solid, 98 mL of water and 28 mL of ethanol (special grade reagent, manufactured by FUJIFILM Wako Pure Chemical Corporation) were added and completely dissolved at 66°C. The liquid was slowly cooled to room temperature and allowed to stand overnight. Precipitated white crystals were obtained by suction filtration and dried overnight under reduced pressure. The yield was 12.2 g.

Synthesis of the compound 3 was performed with reference to a known literature (K. Oertell, C. E. McKenna, M. F. Goodman et al. Biochemistry, 53, 1842, 2014.). To a mixed solvent of 16.8 mL of t-BuOH (manufactured by FUJIFILM Wako Pure Chemical Corporation) and 16.8 mL of water, 953 mg of cytidine-5'-monophosphate and 0.77 mL of morpholine (manufactured by FUJIFILM Wako Pure Chemical Corporation) were added and completely dissolved by stirring while refluxing. 1.83 g of N,N'-dicyclohexylcarbodiimide (Wako primary grade, manufactured by FUJIFILM Wako Pure Chemical Corporation) was dissolved in 19.3 mL of t-BuOH.

One-eighth of the solution was continuously added to the cytidine-5'-monophosphate solution once every 15 minutes. After a total amount thereof was added, the mixture was further refluxed for 2.5 hours. After about 50 mL of water was added to the crude product liquid cooled to room temperature, the generated white solid was removed by suction filtration. Further, filtration was performed with a membrane filter having a pore diameter of 0.45 µm until the filtrate became clear. After the solvent was distilled off under reduced pressure, DMF was added and distilled off again, and the resultant was used for the next reaction without purification.

Synthesis of the compound 4 was performed with reference to a known literature (T. Tanaka et al. yakugakuzasshi, 85, 863, 1965.). 2.00 g of the previously synthesized compound 2 was added and dissolved in 178 mL of water. The solution was passed through a column with an H⁺ type strongly acidic cation exchange resin to remove cyclohexylamine, followed by washing with water to obtain 300 mL of eluate in total. Next, water was distilled off under reduced pressure, and an appropriate amount of DMF was added thereto and distilled off again. DMF was added thereto again for dissolution. Further, the separately synthesized compound 3 was dissolved in DMF, and then both were mixed and stirred overnight at room temperature. At this time, 16.2 mL of DMF including one used for washing was used. The temperature was raised to 60°C on the next day, and the mixture was stirred for 5 hours to complete the reaction. After the solvent was distilled off under reduced pressure, water was added and distilled off again, and the resultant was used for the next reaction without purification.

Synthesis of CDP-ethanolamine was performed with reference to a known literature (T. Tanaka et al. yakugakuzasshi, 85, 863, 1965.). 11.2 mL of water was added to the previously synthesized compound 4 for dissolution. After the atmosphere was replaced with nitrogen, 636 mg of 5 mass% Pd/C (STD type for organic synthesis manufactured by N. E. CHEMCAT) was added, followed by stirring. Next, the atmosphere was replaced with hydrogen, followed by strongly stirring at room temperature for 4 hours. After the atmosphere was replaced with nitrogen again, Pd/C was removed from the reaction solution by suction filtration. Further, remaining Pd/C was removed by a membrane filter having a pore diameter of 0.45 µm. Water was added to the filtrate thus obtained to obtain 1020 mL of a solution containing a cytidine-5'-diphosphate compound. At this time, the solution containing a cytidine-5'-diphosphate compound contained CDP-ethanolamine at a concentration of 1.17 g/L and 5'-CMP at a concentration of 0.0334 g/L (a ratio of 5'-CMP to CDP-ethanolamine 2.9%).

1000 mL of the solution containing a cytidine-5'-diphosphate compound obtained as described above was passed through a column with 150 mL of OH⁻ type PA412 at SV = 1.25 for adsorption. Thereafter, a temperature outside the column was heated to 60°C, and 225 mL of water was passed through the column at SV = 1.10. Finally, while the column was kept at 60°C, 1500 mL of 0.5 mol/L propionic acid aqueous solution was passed through the column at SV = 0.85, and 15.1 mL each of eluate was obtained. The fractions containing CDP-ethanolamine were mixed, and as a result, 151 mL of a solution having a CDP-ethanolamine concentration of 7.50 g/L was obtained. A 5'-CMP concentration of the solution was 0.008 g/L (a ratio of 5'-CMP to CDP-ethanolamine 0.11%). A collection ratio of CDP-ethanolamine was 96.8%. From the results, it is found that the production method of the present invention can also be applied to a CDP-ethanolamine solution obtained by the synthesis method.

### [Comparative Example 4]

140 mL of an aqueous solution containing a CDP trisodium salt (Lot: B12560, manufactured by Combi-Blocks) at a concentration of 6.67 g/L and 5'-CMP at a concentration of 0.43 g/L was prepared, and the aqueous solution was passed through a column with 100 mL of OH⁻ type Marathon A (manufactured by Dow Chemical Company) at SV = 1.25 for adsorption. Next, a temperature outside the column was heated to 40°C, and 150 mL of water was passed through the column at SV = 1.10. Finally, while the column was kept at 40°C, 1500 mL of 0.50 mol/L acetic acid aqueous solution was passed through the column at SV = 0.85, and 15.0 mL each of eluate was obtained. The CDP trisodium salt was not contained in any fraction. From the results, it is found that the production method of the present invention cannot be applied to the CDP trisodium salt.

### [Example 13]

As a solution containing a cytidine-5'-diphosphate compound, 140 mL of an aqueous solution containing CDP-choline at a concentration of 7.09 g/L and 5'-CMP at a concentration of 0.45 g/L (a ratio of 5'-CMP to CDP-choline 6.3%) was prepared, and the aqueous solution was passed through a column with 100 mL of OH⁻ type Marathon A (manufactured by Dow Chemical Company) at SV = 1.25 for adsorption. Next, a temperature outside the column was heated to 40°C, and 150 mL of water was passed through the column at SV = 1.10. Finally, while the column was kept at 40°C, 1000 mL of 0.50 mol/L acetic acid aqueous solution was passed through the column at SV = 0.85, and 10.0 mL each of eluate was obtained. The fractions containing CDP-choline were mixed, and as a result, 110 mL of a solution having a CDP-choline concentration of 8.06 g/L was obtained. The solution did not contain 5'-CMP, and a collection ratio of CDP-choline was 89.3%.

Although the present invention has been described in detail with reference to specific embodiments, it will be apparent to those skilled in the art that various changes and modifications can be made without departing from the spirit and scope of the present invention. The present application is based on an International Application (PCT/JP2021/031429) filed on August 26, 2021, the entire contents of which are incorporated herein by reference. All references cited herein are incorporated in their entirety.

## Claims

1. A method for producing a cytidine-5'-diphosphate compound, the method comprising: the following steps (1) and (2):
(1) a step of bringing a solution containing a cytidine-5'-diphosphate compound into contact with a basic ion exchange resin to adsorb the cytidine-5'-diphosphate compound onto the basic ion exchange resin, and
(2) a step of obtaining an eluate by eluting the cytidine-5'-diphosphate compound adsorbed onto the basic ion exchange resin using an aqueous solution containing an organic acid having 2 or more carbon atoms, wherein the cytidine-5'-diphosphate compound is a cytidine-5'-diphosphate compound having a cationic substituent.

2. The production method according to claim 1, wherein a concentration of the cytidine-5'-diphosphate compound contained in the eluate in the step (2) is equal to or higher than a concentration of the cytidine-5'-diphosphate compound in the solution containing a cytidine-5'-diphosphate compound in the step (1).

3. The production method according to claim 1 or 2, wherein in the step (2), a temperature at which the cytidine-5'-diphosphate compound is eluted from the basic ion exchange resin is 20°C or higher.

4. The production method according to any one of claims 1 to 3, wherein the organic acid having 2 or more carbon atoms is at least one organic acid selected from acetic acid, propionic acid, and butyric acid.

5. The production method according to any one of claims 1 to 4, wherein in the step (2), a concentration of the organic acid contained in the aqueous solution is 0.1 mol/L or more.

6. The production method according to any one of claims 1 to 5, wherein the cytidine-5'-diphosphate compound having a cationic substituent is a compound in which cytidine-5'-diphosphate and a cationic substituent are bonded.

7. The production method according to any one of claims 1 to 6, wherein the cytidine-5'-diphosphate compound having a cationic substituent is a compound in which a cationic substituent is bonded to a β-phosphate group of cytidine-5'-diphosphate.

8. The production method according to any one of claims 1 to 7, wherein the organic acid having 2 or more carbon atoms is a carboxylic acid having 2 or more carbon atoms.

9. The production method according to any one of claims 1 to 8, wherein the cytidine-5'-diphosphate compound having a cationic substituent is at least one selected from cytidine-5'-diphosphate choline and cytidine-5'-diphosphate ethanolamine.
